**Europäisches Patentamt**

(19) **European Patent Office**

**Office européen des brevets**

(11) Veröffentlichungsnummer: **0 066 213**
**B1**

(12) # EUROPÄISCHE PATENTSCHRIFT

(45) Veröffentlichungstag der Patentschrift:
04.09.85

(51) Int. Cl.⁴: **A 61 F 13/20**

(21) Anmeldenummer: **82104450.0**

(22) Anmeldetag: **21.05.82**

(54) **Applikator zum hygienischen Einführen eines Tampons.**

(30) Priorität: **29.05.81 DE 3121364**

(43) Veröffentlichungstag der Anmeldung:
**08.12.82 Patentblatt 82/49**

(45) Bekanntmachung des Hinweises auf die Patenterteilung:
**04.09.85 Patentblatt 85/36**

(84) Benannte Vertragsstaaten:
**AT BE CH DE FR GB IT LI LU NL SE**

(56) Entgegenhaltungen:
**AT - B - 282 815**
**DE - A - 2 054 293**
**US - A - 3 628 533**
**US - A - 3 696 812**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Wiegner, Georg, Breslauer Strasse 35, D-4060 Viersen 11 (DE)**
Erfinder: **Reinwald, Elmar, Dr., Wiesdorfer Strasse 7, D-4000 Düsseldorf (DE)**

EP 0 066 213 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft einen Applikator zum hygienischen Einführen eines Tampons während des Menstruationszyklus der Frau mit einer das Einführen erleichternden, etwa zylindrischen Aussenhülse und einer darin zu verschiebenden Innenhülse zum Ausstossen des Tampons, wobei das vordere Ende der Aussenhülse sich nach vorne verjüngt und einen beim Ausstossen des Tampons zu öffnenden Verschluss darstellt, wobei die Hülsenwandungen aus verschiedene Anforderungen jeweils erfüllenden Einzelschichten zusammengesetzt sind und die Hülsenwandung aus einseitig gestrichenem, hochglänzendem, mit Kunststoff beschichtetem, leicht toilettierbarem Papier besteht, wobei im Sinne einer Erleichterung des Aufstossens des Verschlusses und des Ausbringens des Tampons die Innenfläche der Aussenhülse und die Aussenfläche der Innenhülse aufeinander abgestimmte Reibungskoeffizienten besitzen, wobei die Aussenhülse eine Griffhilfe aufweist und wobei die Aussenhülse, die Innenhülse und der Tampon mit dem Verschluss am Vorderende des Tampons und der Innenhülse an der Rückseite des Tampons ein im wesentlichen geschlossenes System bilden.

An einen Applikator-Tampon, der an sich gegenüber dem Digitaltampon den Vorteil der sauberen und hygienischen Einführung besitzt, werden eine Reihe von nach dem derzeitigen Stand des Wissens in gynäkologischer, umwelt- und markttechnischer Hinsicht zu berücksichtigende Anforderungen gestellt, die sich teilweise widersprechen. Um ein gefahrloses Gleiten beim Einführen in die Scheide zu ermöglichen, soll das Applikatormaterial eine möglichst glatte Oberfläche besitzen. Aus emotionellen Gründen soll sich die Oberfläche jedoch nicht kalt, hart oder kunststoffähnlich anfühlen. Ferner soll der Applikator in der Toilette so aufquellen, dass er weggespült werden kann. Um ein Verletzen durch Einführen des Tampons zu vermeiden, soll das vordere Ende konisch abgerundet sein. Trotzdem soll dieses Ende beim Ausstossen des Tampons so geöffnet werden können, dass der Expulsionsdruck beim Tampon-Ausstoss 500 Gramm nicht überschreitet. Auch darf der Tampon vor Gebrauch nicht aus der Hülle fallen und verschmutzt werden. Der Applikator soll also ein weitgehend geschlossenes System bilden. Schliesslich wäre für ein bequemes Handhaben des Applikators trotz der Forderung nach glatter Oberfläche eine Griffhilfe von Vorteil.

Aus der DE-A-2 054 293 ist ein Applikator zum hygienischen Einführen eines Tampons bekannt, der einen Teil der vorstehend aufgeführten Anforderungen erfüllt.

Weiterhin kann aus der AT-B-282 815, die einen Applikator der eingangs genannten Art beschreibt, entnommen werden, dass es günstig ist, bei einem Applikator-Tampon das Vorderende der äusseren Hülse unter einem geringfügigem Druck leicht entfaltbar auszuführen, wenn der Tampon nach vorne ausgestossen wird. Um dieses Ziel zu erreichen, wird empfohlen, ein hinreichend dünnes Material zu verwenden. Weil bei der Verwendung eines derartigen Materials die ansonsten erforderliche ausreichende Stabilität für die Aussenhülse nicht sichergestellt wird, soll dann am rückwärtigen Ende der Aussenhülse ein Versteifungsring befestigt werden.

Der Erfindung liegt demgegenüber die Aufgabe zugrunde, ein Applikatorsystem zu schaffen, das die vorgenannten Forderungen alle zugleich auf optimale Weise erfüllt. Die erfindungsgemässe Lösung besteht bei dem aus Aussenhülse, Innenhülse und Tampon zusammengesetzten Applikatorsystem der eingangs genannten Art darin, dass der Verschluss aus einem Kranz von am vorderen Ende der Aussenhülse nach Wegstanzen von Teilen zurückbleibenden Lamellen besteht, die im geschlossenen Zustand des Lamellenverschlusses tulpenförmig zusammengelegt sind und einen abgerundeten Kopf bilden, dass an dem Ursprung der Lamellen eine beim Aufstossen des Verschlusses als Scharnier wirkende umlaufende Rille vorgesehen ist und dass die Griffhilfe an der Aussenhülse in Form einer Rillung ausgebildet ist.

Zweckmässige Ausgestaltungen stellen die Konstruktionen gemäss den Unteransprüchen 2 bis 5 dar.

Durch Material, Form und funktionelle Gestaltung sowie Herstellbarkeit des erfindungsgemässen Applikatorsystems wird die gleichzeitige Erfüllung der eingangs genannten Forderungen erreicht. Als Material dient ein einseitig gestrichenes hochglänzendes Papier, das mit Kunststoff beschichtet und insbesondere auch farbig bedruckt sein kann. Die Kunststoffschicht lässt sich mit Hilfe eines Extruders über eine Breitschlitzdüse auf das Papier aufbringen. Die Beschichtung kann dabei beliebig gewählt werden. Den übrigen Forderungen entsprechend ist eine 15 bis 30 g/m$^2$ entsprechende Schichtdicke am vorteilhaftesten. Als Material wird bevorzugt Polyäthylen eingesetzt; doch ist auch die Verwendung von Polypropylen, Polyvinylidenchlorid und anderen sich leicht in der Toilette auflösenden Typen, insbesondere Polyvinylalkohol, günstig.

Durch Verwendung solcher Materialien wird eine Vielzahl von Vorteilen miteinander kombiniert. Zunächst wird die glatte Oberfläche des Kunststoffs mit dem natürlich anmutenden Habitus des Papiers verbunden. Das Material ist vollkommen toilettierbar, leicht zu verformen bzw. zu schneiden und ist in seiner Preisgestaltung im Gegensatz zum reinen Kunststoffapplikator an die Kosten des Naturprodukts Holz gebunden. Durch Einfärben oder Bedrucken der Papierschicht lässt sich die Farbgebung einfach gestalten.

Anhand der schematischen Darstellung eines Ausführungsbeispiels werden weitere Einzelheiten der Erfindung erläutert. Es zeigen:

Fig. 1 die drei Einzelteile des Applikatorsystems, nämlich Aussenhülse, Innenhülse und Tampon;

Fig. 2 einen zusammengesetzten Applikator; und

Fig. 3 einen Applikator mit teilweise durch das Vorderende ausgestossenem Tampon.

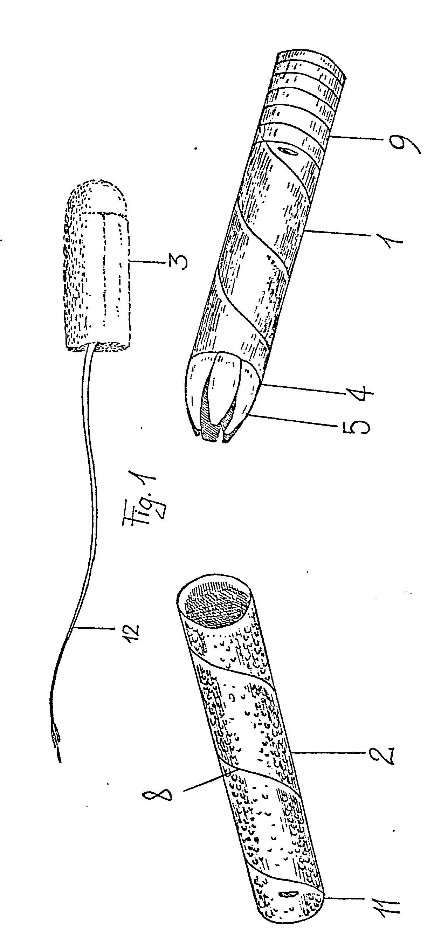

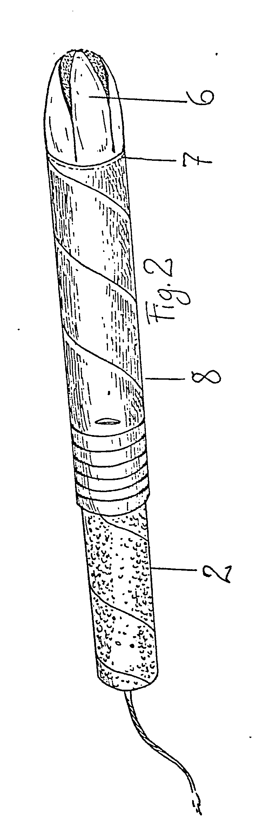

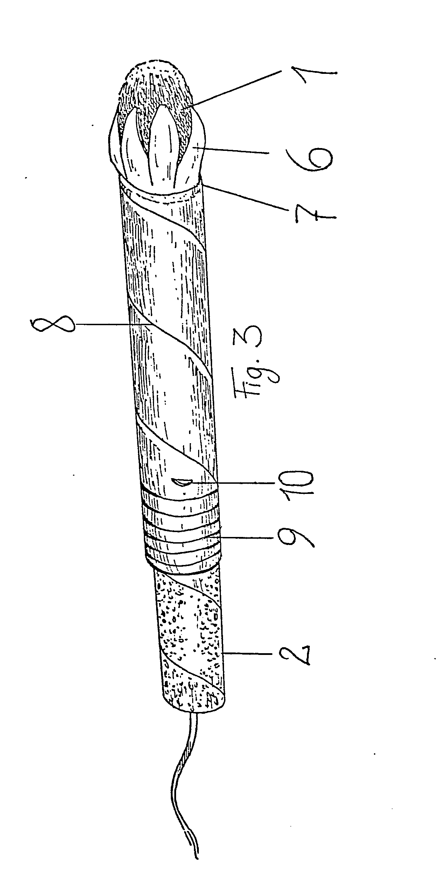

Das in den Figuren 1 bis 3 anhand eines Ausführungsbeispiels dargestellte Applikatorsystem besteht aus Aussenhülse 1, Innenhülse 2 und Tampon 3. Alle drei Teile sind im wesentlichen zylindrisch. Das vordere Ende 4 der Aussenhülse 1 ist bei konisch abgerundeter Formgebung als durch bzw. bei Ausstossen des Tampons 3 zu öffnender Lamellenverschluss 5 ausgebildet. Der Verschluss 5 besteht aus einem Kranz von im verschlossenen Zustand etwa tulpenförmig zusammengelegten Lamellen 6 (vergleiche Fig. 2), an deren Ursprung eine beim Ausstossen als Scharnier wirkende, umlaufende Rille 7 vorgesehen ist. Die – wie aus der Wendellinie 8 ersichtlich – ebenso wie die Innenhülse 2 in der Regel gewickelte Aussenhülse 1 besitzt am rückwärtigen Ende eine Griffhilfe in Form einer Rillung 9 sowie eine Kerbe 10 zum Fixieren gegenüber der Innenhülse 2. Der Tampon 3 wird in der Aussenhülse 1 auf der Vorderseite durch den Lamellenverschluss 5 und auf der Rückseite durch das umgebördelte Ende 11 der Innenhülse 2 festgehalten. Durch die Innenhülse 2 erstreckt sich der Ziehfaden 12 des Tampons 3.

Durch den Einsatz des beschriebenen Materials kann eine äusserst funktionelle Formgebung erzielt werden. Das ist besonders am Beispiel des als Lamellenverschluss 5 ausgebildeten Applikator-Rundkopfes ersichtlich. Beim Herstellen des Rundkopfes werden, wie unten noch näher beschrieben wird, am vorderen Ende der Aussenhülse 1 Teile so weggestanzt, dass sich der Kranz von Lamellen 6 ergibt. Dieser Kranz lässt sich bei Auswahl passender Materialeigenschaften des gegebenenfalls mehrschichtigen Papiers zu einem tulpenförmigen Verschluss verformen.

Damit sich der geformte Lamellenverschluss 5 beim Ausstossen des Tampons 3 leicht öffnen lässt, wird am Ursprung der Lamellen 6 die Rille 7 umlaufend um die Aussenhülse 1 vorgesehen. Diese Rille 7 wirkt auf die Lamellen 6 wie ein Scharnier, derart, dass bei relativ leichtgängig aufzustossendem Lamellenverschluss ein problemloses Ausbringen des Tampons 3 bei geringem Expulsionsdruck, möglich ist.

Das Ausstossen des Tampons 3 wird auch durch sorgfältiges Abstimmen der Reibungskoeffizienten von Innenpapier bzw. -schicht der Aussenhülse 1 und Aussenpapier bzw. -schicht der Innenhülse 2 erleichtert. Das Abstimmen der Reibungskoeffizienten unabhängig von den meisten anderen Forderungen und Funktionen ist möglich, weil die Hülsenwand aus mehreren Schichten aufgebaut sein kann. Es lassen sich daher unabhängig voneinander an Aussen- und Innenseite der Hülsen 1 und 2 sehr verschiedenartige Materialeigenschaften einstellen bzw. Forderungen erfüllen.

Der erfindungsgemässe Applikator lässt sich als geschlossenes System ausbilden, da der Tampon auf dem vorderen Ende durch den Lamellenverschluss 5 und auf der Rückseite durch die Bördelung 11 der Innenhülse 2 festzuhalten ist. Die Innenhülse 2 kann über die Kerbe 10 an der Aussenhülse 1 fixiert werden. Beim Ausstossen wird diese Fixierung überwunden.

Der neue Applikator kann – nach gesonderter Wicklung – zusammen mit dem Tampon auf ein und derselben Maschine in seine endgültige Form gebracht werden. In einem Ausführungsbeispiel wird die vorgefertigte Hülse auf einen beheizten Innendorn geschoben, wobei mehrere solcher Dorne auf einer sich getaktet drehenden Scheibe sitzen können. In dem Ausführungsbeisiel fährt gegen diesen Dorn zunächst eine Stanze, die Segmente so ausschneidet, dass ein Kranz von Lamellen zurückbleibt. An der nächsten Station werden am Ursprung der Lamellen 6 und am gegenüberliegenden Ende die entsprechenden Rillungen 7, 9 angebracht. Schliesslich werden in der letzten Station der Scheibe die Lamellen 6 mit Hilfe eines beheizten Werkzeugs zu dem bereits beschriebenen tulpenförmigen Lamellenverschluss 5 umgebördelt. Diese fertige Aussenhülse 1 wird nun in eine Trommel eingegeben, in der Aussenhülse 1, Tampon 3 und Innenhülse 2 hintereinander liegen. Mit Hilfe eines Stössels können dort die drei Einzelelemente ineinandergeschoben und mit Hilfe einer Nadel kann durch Kerben 10 der Zusammenhalt gesichert werden.

Bezugszeichenliste
  1 = Aussenhülse
  2 = Innenhülse
  3 = Tampon
  4 = vorderes Ende
  5 = Lamellenverschluss
  6 = Lamellen
  7 = Rille
  8 = Wendellinie
  9 = Rillung
  10 = Kerbung
  11 = Bördelung
  12 = Ziehfaden

**Patentansprüche**

1. Applikator zum hygienischen Einführen eines Tampons (3) während des Menstruationszyklus der Frau mit einer das Einführen erleichternden, etwa zylindrischen Aussenhülse (1) und einer darin zu verschiebenden Innenhülse (2) zum Ausstossen des Tampons (3), wobei das vordere Ende (4) der Aussenhülse (1) sich nach vorne verjüngt und einen beim Ausstossen des Tampons (3) zu öffnenden Verschluss (5) darstellt, wobei die Hülsenwandungen aus verschiedene Anforderungen jeweils erfüllenden Einzelschichten zusammengesetzt sind und die Hülsenwandung aus einseitig gestrichenem, hochglänzendem, mit Kunststoff beschichtetem, leicht toilettierbarem Papier besteht, wobei im Sinne einer Erleichterung des Aufstossens des Verschlusses (5) und des Ausbringens des Tampons (3) die Innenfläche der Aussenhülse (1) und die Aussenfläche der Innenhülse (2) aufeinander abgestimmte Reibungskoeffizienten besitzen, wobei die Aussenhülse (1) eine Griffhilfe (9) aufweist und wobei die Aussenhülse (1), die Innenhülse (2) und der Tampon (3) mit dem Verschluss (5) am Vorderende des Tampons (3) und der Innenhülse (2) an der Rückseite des Tampons (3) ein im wesentlichen geschlossenes System bilden, dadurch gekennzeichnet, dass der Verschluss (5) aus einem Kranz von am vorderen Ende (4) der Aussenhülse (1) nach Wegstanzen von Teilen zurückbleibenden Lamellen (6) besteht, die im geschlossenen Zustand des Lamellenverschlusses (5) tulpenförmig zusammengelegt sind und einen abgerundeten Kopf bilden, dass an dem Ursprung der Lamellen (6) eine beim Aufstossen des Verschlusses als Scharnier wirkende umlaufende Rille (7) vorgesehen ist und dass die Griffhilfe an der Aussenhülse (1) in Form einer Rillung (9) ausgebildet ist.

2. Applikator nach Anspruch 1, dadurch gekennzeichnet, dass die Kunststoffbeschichtung des Papiers aus Polyäthylen, Polypropylen, Polyvinylidenchlorid, Polyvinylalkohol oder ähnlichem, leicht in Wasser aufzulösendem Material besteht.

3. Applikator nach Anspruch 1 oder 2, dadurch gekennzeichnet, dass die Innenhülse (2) mit Hilfe einer Kerbe (10) an der Aussenhülse (1) fixiert ist.

4. Applikator nach einem oder mehreren der Ansprüche 1 bis 3, dadurch gekennzeichnet, dass die Hülsenaussenfläche farbig bedruckt ist.

5. Applikator nach einem oder mehreren der Ansprüche 1 bis 4, dadurch gekennzeichnet, dass die Innenhülse (2) mit einem umgebördelten Längsende (11) versehen ist.

## Revendications

1. Applicateur pour l'insertion hygiénique d'un tampon (3), pendant le cycle de menstruation de la femme, comportant un manchon extérieur à peu près cylindrique (1), facilitant l'insertion, et un manchon intérieur (2), que l'on peut faire coulisser dans le précédent pour l'expulsion du tampon (3), l'extrémité antérieure (4) du manchon extérieur (1) rétrécissant vers l'avant et constituant une fermeture (5) qu'il faut ouvrir lors de l'expulsion du tampon (3), les parois des manchons étant composées de différentes couches, remplissant chacune différentes exigences, formées de papier trés brillant et revêtu sur une face de matière synthétique, facilement jetables dans les toilettes, la surface intérieure du manchon extérieur (1) et la surface extérieure du manchon intérieur (2) présentant, dans le sens d'une facilitation de l'expulsion de la fermeture (5) et de l'extraction du tampon (3), des coefficients de frottement harmonisés entre eux, le manchon extérieur (1) présentant une extrémité de prise (9) et le manchon extérieur (1), le manchon intérieur (2) et le tampon (3) formant, avec la fermeture (5) de l'extrémité antérieure du tampon (3) et du manchon intérieur (2) à l'arrière du tampon (3), un système pratiquement fermé, applicateur caractérisé par le fait que la fermeture (5) est constituée d'une couronne de lamelles (6) restant à l'extrémité antérieure (4) du manchon extérieur (1) après le découpage des parties, ces lamelles (6), dans l'état fermé de la fermeture (5), étant réunies en forme de tulipe et formant une tête arrondie, une gorge circonférentielle (7) étant prévue à l'origine des lamelles (6) pour jouer le rôle de charnière lors de l'expulsion de la fermeture et l'extrémité de prise (9) du manchon extérieur (1) étant constituée sous la forme d'un ensemble cannelé.

2. Applicateur selon la revendication 1, caractérisé par le fait que le revêtement de matière synthétique du papier est constitué de polyéthylène, de polypropylène, de polychlorure de vinylidène, d'alcool polyvinylique ou de matière analogue facilement soluble dans l'eau.

3. Applicateur selon l'une ou l'autre des revendications 1 ou 2, caractérisé par le fait que le manchon intérieur (2) est fixé au manchon extérieur (1) à l'aide d'un cran (10).

4. Applicateur selon l'une quelconque des revendications 1 à 3, caractérisé par le fait que la surface extérieure du manchon extérieur est imprimée en couleur.

5. Applicateur selon l'une quelconque des revendications 1 à 4, caractérisé par le fait que le manchon intérieur (2) est muni d'une extrémité longitudinale (11) rabattue.

## Claims

1. Applicator, for the hygienic insertion of a tampon (3) during the menstruation cycle of the woman, with an about cylindrical outer sleeve (1), which facilitates the introduction, and an inner sleeve (2) to be displaced therein for the ejection of the tampon (3), wherein the forward end (4) of the outer sleeve (1) narrows forwardly and represents a closure (5) to be opened during the ejection of the tampon (3), wherein the sleeve walls are composed of individual layers each respectively fulfilling different requirements and the sleeve wall consists of one-sidedly painted, highly glossy paper, which is coated with synthetic material and easily disposable in a toilet, wherein the internal surface of the outer sleeve (1) and the outer surface of the inner sleeve (2) possess coefficients of friction matched one to the other in the sense of a facilitation of pushing the closure (5) open and bringing the tampon (3) out, wherein the outer sleeve (1) displays a gripping aid (9) and wherein the outer sleeve (1), the inner sleeve (2) and the tampon (3) form a substantially closed system with the closure (5) at the front end of the tampon (3) and the inner sleeve (2) at the rear side of the tampon (3), characterised thereby, that the closure (5) consists of a ring of laminae (6), which after the stamping away of parts remain behind at the forward end (4) of the outer sleeve (1) and in the closed state of the laminae closure (5) are laid together in tulip shape and form a rounded head, that an encircling groove (7), which acts as hinge during the pushing-open of the closure, is provided at the root of the laminae (6) and that the gripping aid is formed at the outer sleeve (1) in the form of a grooving (9).

2. Applicator according to claim 1, characterised thereby, that the synthetic material coating of the paper concists of polyethylene, polypropylene, polyvinylidene chloride, polyvinyl alcohol or similar material easily soluble in water.

3. Applicator according to claim 1 or 2, characterised thereby, that the inner sleeve (2) is fixed with the aid of a notch (10) at the outer sleeve (1).

4. Applicator according to one or more of the claims 1 to 3, characterised thereby, that the outer lseeve surface is printed on in colour.

5. Applicator according to one or more of the claims 1 to 4, characterised thereby, that the inner sleeve (2) is provided with a beaded-over longitudinal end (11).

Fig. 1

Fig. 2

fig. 3